# EUROPEAN PATENT SPECIFICATION

(11) **EP 2 914 754 B1**
(45) Date of publication and mention of the grant of the patent: **04.01.2017**
(21) Application number: 14796307.8
(22) Date of filing: 29.09.2014
(51) Int. Cl.: C12N 1/20, C12R 1/225

(54) **NEW STRAIN OF LACTOBACILLUS DELBRUECKII BACTERIA AND ITS USE FOR THE PRODUCTION OF BEE POLLEN**
LACTOBACILLUS DELBRUECKII STAMM UND DESSEN VERWENDUNG FÜR DIE HERSTELLUNG VON BIENENPOLLEN
SOUCHE DE LACTOBACILLUS DELBRUECKII ET SON UTILISATION POUR LA PRODUCTION DE POLLEN D'ABEILLE

(30) Priority: 20.12.2013 PL 40662213
(43) Date of publication of application: 09.09.2015
(73) Proprietor: Instytut Biotechnologii Przemyslu Rolno-Spozywczego, 02-532 Warszawa (PL)
(72) Inventor: MISIEWICZ, Anna, 02-156 Warszawa (PL); KIELISZEK, Marek, 21-010 Leczna (PL); CZARNIAK, Katarzyna, 03-986 Warszawa (PL)
(74) Representative: Witek, Rafal
(86) International application number: PCT/PL2014/050061
(87) International publication number: WO 2015/093997

(56) References cited:
- WO-A1-2008/043161
- WO-A1-2008/136730
- WO-A1-2010/058294
- VASQUEZ ALEJANDRA ET AL: "The lactic acid bacteria involved in the production of bee pollen and bee bread", JOURNAL OF APICULTURAL RESEARCH, INTERNATIONAL BEE RESEARCH ASSOCIATION, CARDIFF, GB, [Online] vol. 48, no. 3, 1 July 2009 (2009-07-01), pages 189-195, XP008174033, ISSN: 0021-8839

## Description

The subject of the invention is a new strain of Lactobacillus delbrueckii bacteria. The invention finds use in the food industry.

*Lactobacillus delbrueckii* strains are known which hydrolyse sugars producing lactic acid, however they are' not able to hydrolyse sugars in worts with a high osmotic pressure. For most bacteria, higher osmotic pressure is a factor restricting growth. Honey wort high osmotic pressure, as well as the presence of anti-fungal and anti-bacterial compounds results in the growth of most microorganisms being restricted. Nevertheless Lactobacillus species have been isolated from bee pollen (Vasquez A. and Olofsson T.C., Journal of Apicultural Research, vol. 48, no. 3, 1 July 2009, pages 189-195).

In accordance with the classical Orla-Jensen systematics, *Lactobacillus* species bacteria are able to produce lactic acid disaccharides and monosaccharides in the form of D (-) and L(-) isomer. There is a need for additional lactic acid bacteria strains in the state of knowledge pertaining to useful properties of certain types of lactic acid fermentation bacteria able to hydrolyse sugars in high osmotic pressure producing lactic acid. The aim of the invention was to obtain a new strain of *Lactobacillus* genus able to survive in high osmotic pressure and a method for producing bee pollen with the use of that strain which will be which will have a naturally long shelf life due to the presence of lactic acid.

The first subject of the invention is the *Lactobacillus delbrueckii* strain of bacteria deposited at the Culture Collection of Industrial Micro-organisms in The Institute of Agricultural and Food Biotechnology in Warsaw under number KKP 1645 p.

The second subject of the invention is the use of the strain defined in the first subject of the invention to produce bee pollen.

The third subject of the invention is bee pollen characteristic in that it contains the strain of bacteria defined in the first subject of the invention.

The *Lactobacillus delbrueckii* KKP 1645 p strain of bacteria was obtained by isolating a bacteria monoculture from natural bee pollen extracted from hive frames in the final stages of fermentation. The bee pollen was extracted from honeycomb cells and places in 10 cm³ of liquid MRS bullion. It was incubated for 48h at 25°C, 30°C and 37°C in strictly anaerobic and aerobic conditions. All tested samples were diluted tenfold, out of which 100 µl of bacteria cultures were taken onto an MRS agar solid growth media. It was incubated for 24h-72h at 25°C, 30°C and 37°C in strictly anaerobic and aerobic conditions. Then the bacteria were subject to multiple selections. The selection and the ensuing adaptation to new growing conditions took place on growth media with glucose, fructose, starch and sucrose as the only sources of carbon and in honey warts with density of 7, 15, 30, 40, 45 and 50° Blg.

As a result a new strain was developed with the following characteristics:
- morphological - bacteria colonies growing on solid MRS agar growth medium in strictly anaerobic conditions are oval in shape, slightly convex, milky colour with regular edges and approx. 1mm in size; young colonies are creamy and older ones take on a rusty hue. Under a microscope the bacteria take on the form of single or groups of short, kinked rods They do not produce endospores. They are Gram +, catalase-negative.
- physiological and biochemical - optimal temperature for strain growth is 30°C - 37°C. No growth occurs in threshold temperatures of 25°C and 45°C.

It was determined that the new strain, similarly to other *Lactobacillus delbrueckii* species strains, according to bioMerieux API test ((API 50 CHL) is able to ferment the following sugars: D-glucose, D-fructose, sucrose, trehalose, gluconate. Furthermore, unexpectedly it turned out that this strain ferments starch.

In accordance with the adopted aim of the invention the new strain of *Lactobacillus delbrueckii* KKP 1645 p bacteria has very high osmotic resistance, i.e. the ability to survive in concentrations up to 50°Blg. Unexpectedly it turned out that this strain hydrolyses starch with the production of lactic acid. The new *Lactobacillus delbrueckii* KKP 1645 p strain has no equivalent among the known *Lactobacillus* genus to decompose starch and honey wort.

Example applications of the invention are shown on the diagram where fig. 1 shows the growth of *Lactobacillus delbrueckii* KKP 1645 p bacteria when growing on honey worts, fig. 2 the course of a pollen and honey wort control test in restricted oxygen access conditions and temperature of 30 °C, fig. 3 depicts the course of pollen with honey wort fermentation with the sue of 50% inoculum *L*. *delbrueckii* subsp. *delbrueckii* no. 2 KKP 1645 p bacteria in restricted oxygen access conditions and temperature of 30 °C

### Example I

The scope of tests included growth of live *Lactobacillus delbrueckii* KKP 1645 p bacteria in fluid MRS medium with 2% fructose over 24h at 25°C, 30°C, 37°C and 45°C [table 1]. In this test no growth of lactic acid fermentation bacteria growth was observed only at 25°C and 45°C.

**Table 1**

| Effects of temperature on growth if *Lactobacillus delbrueckii* KKP 1645 p bacteria | |
|---|---|
| Incubation temperature | KKP 1645 |
| 25°C | - |
| 30°C | + |
| 37°C | + |
| 45°C | - |

### Example II

The scope of tests included growth of live *Lactobacillus delbrueckii* KKP 1645 p bacteria in fluid MRS medium with 2% fructose containing various additives of NaCl (0.5-12 %) over 24h at a temperature of 37°C[table2]. This test did not show a significant effect of an NaCl on growth of lactic acid, fermentation bacteria.

**Table 2**

| Effects of NaCl concentrations of *Lactobacillus delbrueckii* KKP 1645 p strain | |
|---|---|
| NaCl concentration (%) | KKP 1645 |
| 0.5 | + |
| 5 | + |
| 8 | + |
| 10, | + |
| 12 | + |

### Example III

The scope of tests included growth of live *Lactobacillus delbrueckii* KKP 1645 p bacteria in fluid MRS medium with 2% fructose of pH 4.0, 5.5, 6.5, 7.5, 8.0 over 24h at a temperature of 37°C [table3]. In this test no growth of lactic acid fermentation bacteria growth was observed only at pH 4.0.

**Table 3**

| Effects of pH of *Lactobacillus delbrueckii* KKP 1645 p strain | |
|---|---|
| pH | KKP 1645 |
| 4 | - |
| 5.5 | + |
| 6.5 | + |
| 7.5 | + |
| 8 | + |

### Example IV

Growth of live *Lactobacillus delbrueckii* KKP 1645 p bacteria. in MRS bullion with 2% fructose and placed for 24h at a temperature of 37°C.

The ability to produce lactic acid was determined by conducting a fermentation process on fluid media containing glucose, fructose, sacarose, starch, lactose as the only source of carbon on honey worts 7°Blg, 15°Blg, 30°Blg at a temperature of 37°C.

Media for lactic acid biosynthesis were grafted with 10% cultures. The lactic acid biosynthesis process in a rotating shaker at 150 rpm in flat bottom flasks volume 500 cm³ containing 100 cm³ fermentation medium. The growth temperature was 37°C for 72h.

Table 4 presents the results of the conducted lactic acid biosynthesis by the new *Lactobacillus delbrueckii* KKP 1645 p. strain.

**Table 4**

| Results of the conducted lactic acid biosynthesis (72h) by the *Lactobacillus delbrueckii* KKP 1645 p strain. | | | |
|---|---|---|---|
| Medium | Amount of lactic acid in g/100 cm³ of fluid | | Reducing sugars expressed -in glucose [%] |
| | D(+) form | L (-) form | |
| Fructose | 2.9 | 0.8 | 0.1 |
| Glucose | 3.7 | 0.3 | 0.17 |
| Sucrose | 3.8 | 0.2 | 0.75 |
| Starch | 2.1 | 1.8 | 0.1 |
| 7° Blg honey wort | 4.5 | 0.14 | 1.98 |
| 15° Blg honey wort | 4.3 | 0.22 | 2.11 |
| 30° Blg honey wort | 3.9 | 0.31 | 11.62 |

### Example V

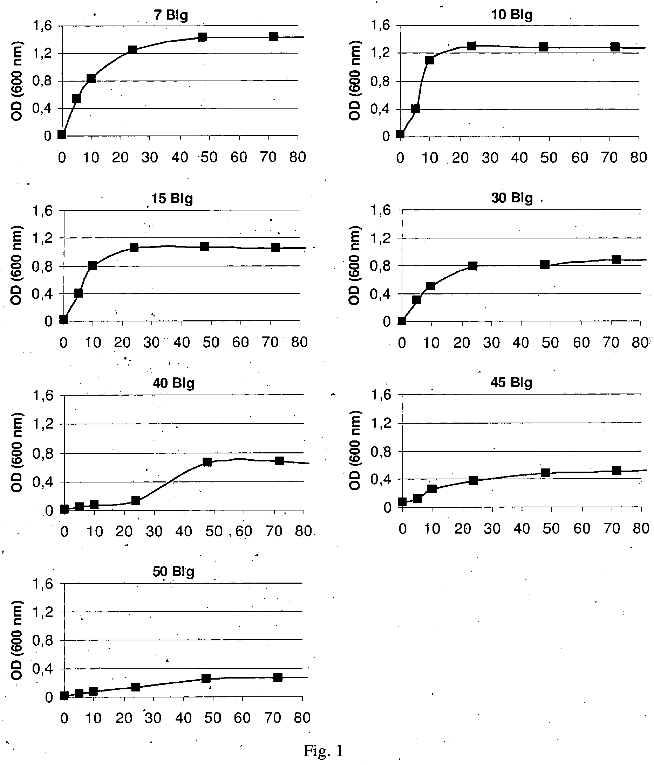

The effects of worts on the growth of bacteria in OD (optical density system was determined. The propagation of bacteria was conducted after grafting the fluid growing medium 1 cm³ 24 hours of *Lactobacillus delbrueckii* KKP 1645 p bacteria growth. The determination of optical density was carried out in order to determine the growth of bacterial cell on 7, 15, 30, 40, 45 and 50° Blg honey wort [table 5]. OD measurements were carried out using a spectrophometer at wave length λ=550 nm. Comparing with known *Lactobacillus* genus strains, biomass growth for *Lactobacillus delbrueckii* KKP 1645 p strain on honey worts is significantly higher. The new *Lactobacillus delbrueckii* KKP 1645 p strain is tolerant to higher concentrations of sugars - has high osmotic tolerance as compared with other lactic acid fermentation bacteria. Growth if *Lactobacillus delbrueckii* KKP 1645 p bacteria on honey worts is shown on fig. 1.

### Example VI

Determining antagonistic *Lactobacillus delbrueckii* KKP 1645 p strain properties.

The inter strain antagonism test was carried out by the drip method (Klewicka E. et al. 1999. Antagonistic activity of Lactobacillus acidophilus lactic acid fermentation bacteria. Food. Science. Technology. Quality). It entails even growth of the control and tested strains. The group of control microorganisms constituted *Bacillus cereus* KKP 358, *Listeria monocytogenes* KKP 1170, *Salmonella* sp. KKP 996, *Pseudomonas aeruginosa* KKP 161, *Escherichia coli* KKP 364, *Serratia liquefaciens* KKP 1665, *Staphylococcus aureus* KKP 1497, *Staphylococcus saprophyticus* KKP 1493. These bacteria originated from Culture Collection of Industrial Micro-organisms in IAFB.

Results of antagonistic *Lactobacillus delbrueckii* KKP 1645 p strain properties tests are shown in table 5.

**Table 5**

| Antagonistic reaction of *Lactobacillus delbrueckii* KKP 1645 p strain with respect of control bacteria. | |
|---|---|
| Control strain | Diameter of inhibition zone (in mm) |
| *Serratia liquefaciens* KKP 1665 | 19 |
| *Escherichia coli* KKP 364 | 19 |
| *Salmonella sp.* KKP 996 | 19 |
| *Listeria monocytogenes* KKP 1170 | 23 |
| *Pseudomonas aeruginosa* KKP 161 | 17 |
| *Staphylococcus aureus* KKP 1497 | 20 |
| *Staphylococcus saprophyticus* KKP 1493 | 17 |
| *Bacillus cereus* KKP 358 | 21 |

*Lactobacillus delbrueckii* KKP 1645 p cultures were applied to solid MRS agar growth media in the form of 5 µl drops and after 24 h- incubation at 37°C the surface was filled with a lawn containing 10⁸ jtk/ml of control strain. Then the media were incubated again for 24h at 37°C. After incubation control bacteria growth inhibition zone occurred around the lactic acid bacteria colonies with its diameter in mm taken as the measure of antagonism.

### Example VII

In order to characterise selected lactic acid *Lactobacillus delbrueckii* KKP 1645 p bacteria, an antibiotics resistance analysis was carried out by KB test using MRS Agar growth medium and Blickfeldt solution with CaCO₃ [table 6].

**Table 6**

| *Lactobacillus delbrueckii* KKP 1645 p strain antibiotic resistance test results (diameter of inhibition zone in mm) | | |
|---|---|---|
| Antibiotic | MRS medium | Blickfeldtmedium with CaCO3 |
| Ampicillin | 40 | 37 |
| Penicillin | 48 | 48 |
| Streptomycin | 0 | 17 |
| Erythromycin | 34 | 37 |

### Example VIII

In order to test the possibility of using the strain of bacteria in accordance with the invention to produce bee pollen a fermentation with the use of it was carried out. 10 % or 50 % strain inoculum in the form of a 48h growth was added to worts with pollen. The number of bacteria in the added inoculum was in the region of 10⁶-10⁷ jtk/ml. At the same time fermentation in control samples was carried out with wort density of 7, 14 i 30 °Blg, containing solely natural pollen microflora.

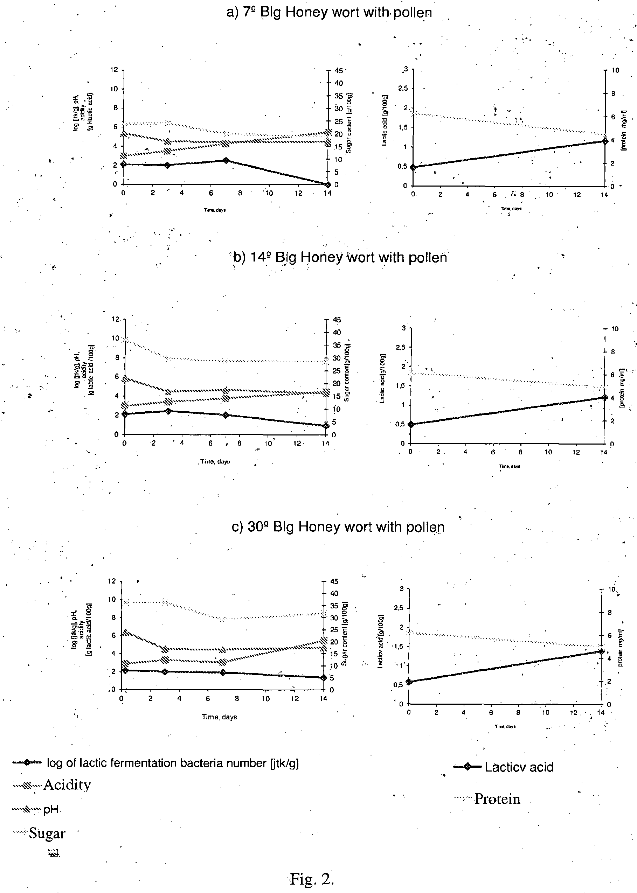

The course of control sample pollen fermentation is depicted in fig. 2. All three samples with density 7, 14 i 30 °Blg a reduction in the number of lactic acid fermentation bacteria occurred from 10² do 10¹ jtk/g in media of 14 and 30 °Blg density and a total growth inhibition of lactic acid bacteria in the 7 °Blg density medium. The most lactic acid, 1.39 g/100g, was obtained at density 30°Blg, and the least at 7 °Blg. In all active samples, just as previously a gradual rise in acidity was determined - the largest in 7 °Blg density medium up to 5.5 g lactic acid / 100g. Also the amount of sugars in media regardless of the density of the fermentation medium as well as protein in supernatant decreased.

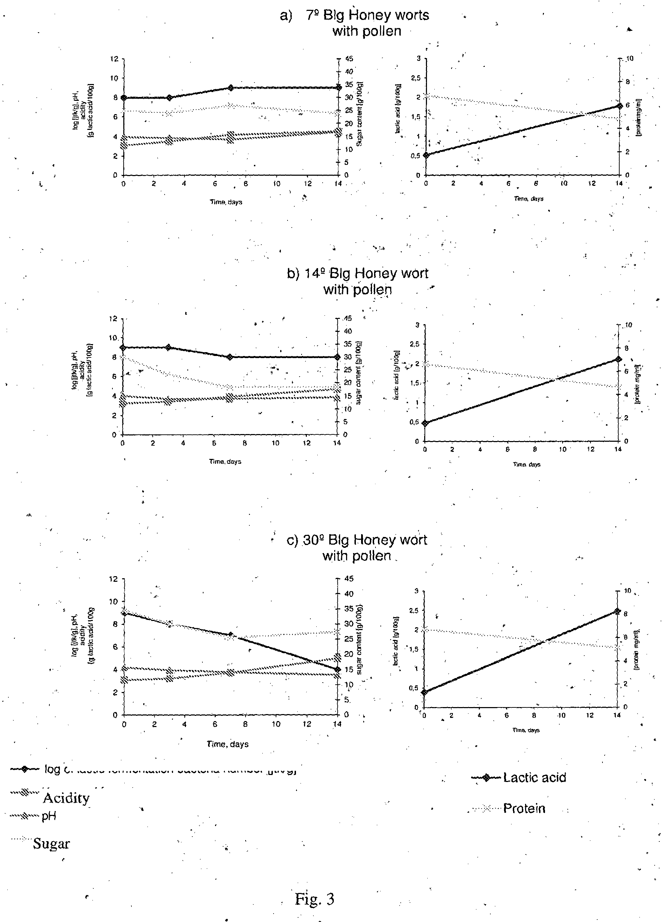

Fig. 3 shows the fermentation progress of pollen and honey wort mixture over a 14 day fermentation with 50% inoculum containing the strain as described by the invention. 10¹⁰ jtk/ml density inoculum was used to graft the medium. In 7, 14 °Blg density media over the entire fermentation period the number of lactic acid bacteria remained at a similar level. A clear reduction in lactic acid fermentation bacteria was evident in 30 °Blg: density medium. Increasing the amount of inoculum from 10 % to 50 % no increase ion? the final lactic acid content was observed. Similar values were obtained, whereas the biggest for *Lactobacillus delbrueckii* subsp. *delbrueckii* nr 2 KKP 1645 (30 °Blg) strain was 2.48 g/ 100 g. Similarly to previous fermentations the amount of sugars and protein decreased.

## Claims

1. New strain of *Lactobacillus delbrueckii* bacteria deposited at the Culture Collection of Industrial Micro-organisms in The Institute of Agricultural and Food Biotechnology in Warsaw under number KKP 1645 p.

2. The use of the strain referred to in claim 1 to produce bee pollen.

3. Bee pollen characteristic in that it contains the strain of bacteria defined in claim 1.

## Patentansprüche

1. Neuer Stamm von *Lactobacillus delbrueckll*-Bakterien, hinterlegt bei der Kultursammlung für industrielle Mikroorganismen Im Institute of Agricultural and Food Biotechnology in Warschau unter der Nummer KKP 1645 p.

2. Verwendung des in Anspruch 1 genannten Stamms zur Herstellung von Bienenbrot.

3. Bienenbrot, **dadurch gekennzeichnet, daß** es den In Anspruch 1 definierten Bakterienstamm enthält.

## Revendications

1. Nouvelle souche de la bactérie *Lactobacillus delbrueckll* déposée au Culture Collection of Industrial Micro-organisms à l'Institut de Biotechnologie Agricole et Alimentaire à Varsovie sous le numéro KKP 1645 p.

2. Utilisation de la souche répertoriée à la revendication 1, pour la production de pollen d'abeille.

3. Pollen d'abeille **caractérisé en ce qu'**il contient la souche bactérienne telle que définie à la revendication 1.
